Europäisches Patentamt

European Patent Office

Office européen des brevets

(19)

(11) Veröffentlichungsnummer: **0 214 320**
**A1**

(12) ## EUROPÄISCHE PATENTANMELDUNG

(21) Anmeldenummer: 85111028.8

(22) Anmeldetag: 02.09.85

(51) Int. Cl.⁴: **C12M 1/00**

Ein Antrag gemäss Regel 88 EPÜ auf Berichtigung von Seite 3 und der Zeichnungen liegt vor. Über diesen Antrag wird im Laufe des Verfahrens vor der Prüfungsabteilung eine Entscheidung getroffen werden (Richtlinien für die Prüfung im EPA, A-V, 2.2).

(43) Veröffentlichungstag der Anmeldung:
**18.03.87 Patentblatt 87/12**

(84) Benannte Vertragsstaaten:
**AT BE CH DE FR GB IT LI LU NL SE**

(71) Anmelder: **Weber, Fritz**
**Schulstrasse 42**
**D-7311 Bissingen/Teck(DE)**

(72) Erfinder: **Weber, Fritz**
**Schulstrasse 42**
**D-7311 Bissingen/Teck(DE)**

(54) **Neue Verfahrenstechnik für betriebsfähige Biogas-Dunggäranlagen (naturkonformes Recycling), welche alle organischen Abfallstoffe aufzunehmen vermag.**

(57) Zu zwei anaeroben Gärbehältern, nämlich dem Hauptgärraum und dem Nachgärraum, werden mindestens vier Kammern als Gärrottebehälter angebaut. Diese Gärrottebehälter sind so angeordnet, daß sie für die beiden vorgenannten Gärbehälter gleichzeitig als Wärmeschutz und während des aeroben Rotteprozesses als Wärmequelle dienen.

EP 0 214 320 A1

Neue Verfahrenstechnik für betriebsfähige Biogas-Dunggäranlagen (naturkonformes Recycling), welche alle organischen Abfallstoffe aufzunehmen vermag. Mit Trennung von Feststoffen und Gärflüssigkeit zu nährstoffreichem Festmist und emmisssionsfreier Gärgülle durch Einschaltung von Pilzkulturen zur Aufschließung von Stroh und Rohfaser in einem 5stufigen Prozeß und in Anlagesystemen für alle Betriebsgrößen und Temperaturbereiche. Antrag auf Erteilung eines VERFAHRENSPATENTES.

Inhaltsübersicht

1. Stand der Technik
2. Aufgabenstellung und deren Lösung dargestellt anhand der neuen Verfahrenstechnik
2.1. Die fünf Stufen des Verfahrens
3. Patent-Anspruchsbegründung mit Angabe der technischen Merkmale des neuen Verfahrens
4. Die naturwissenschaftlichen Grundlagen als Anspruchsbegründung für ein Verfahrenspatent
5. Die verschiedenen Temperaturbereiche der Gärungsvorgänge
5.1. Physophiler Gärbereich
5.2. Mesophiler Gärbereich
5.3. Hochgärtemperaturbereich
6. Betriebs-und volkswirtschaftliche Nutzenberechnungen
6.1. Wirtschaftlichkeit
6.1.1. Ausgangssituation
6.1.2. Nährstoffbilanz
6.2. Volkswirtschaftlicher Wert
7. Ökologische Folgerungen
7.1. Korrektur der Lehrmeinung in der Agrikulturchemie
7.2. Raum, Zeit, Gewicht und Energiebilanz mit dem daraus resultierenden Transportproblem
7.3. Der Irrweg des Sozialismus einer technologisierten Landwirtschaft in Sowchosen und Kolchosen
8. Zusammenfassung
9. Literaturverzeichnis und Zeichnungen

## 1. Stand der Technik

Vom Stand der Technik ausgehend, gelten alle Biogas-und ähnliche Dungvergärungen im reinen Flüssigkeitsbereich. Aus Gründen der Arbeitsrationalität ergab sich der Weg zu Flüssiggülle, Treibmist und ähnlichen "stinkenden" technischen Errungenschaften.

Zum Stand der Technik, seit meiner Patentrücknahme, zählen auch meine Patentanmeldungen P 27 11 146.2 und P 30 07 064.5, sowie meine Zurücknahmeerklärung vom 30.09.1981.

## 2. Aufgabenstellung und deren Lösung dargestellt anhand der neuen Verfahrenstechnik

Im neuen Verfahren wird nun in drei weiteren Stufen durch Einbau von Gärottebehältern mit technischem Zubehör die seither nicht erreichbare Aufschließung von Stroh und ähnlichen Substanzen durch einen aeroben Verpilzungsprozeß der entnommenen Schwimmdecke erreicht.

Die erste Phase einer aeroben Rottegärung vollzieht sich in den einzelnen Gärrottebehältern als Prozeß, der durch die Beigabe von kleineren Mengen frischer organischer Substanz eine Eigenerwärmung erreicht.

Diese Eigenerwärmung ist vorteilhaft für die Entfaltung von Pilzkulturen und ist als Wärmequelle für die Gesamtanlage von wesentlichem Nutzen.

Die Dauer dieses aeroben Rotteprozesses hängt von verschiedenen Faktoren ab, wie z.B. der Beschaffenheit der eingebrachten organischen Substanzen und ihrem prozentualen Anteil gegenüber dem aeroben vergorenen Schwimmdeckenmist - (fast ausschließlich Bakterienkultur).

In der Praxis muß die Dauer dieses Vorganges für jede Anlage individuell erforscht und festgelegt werden, weil jeder landwirtschaftliche Betrieb eine eigene Betriebsindividualität besitzt.

Dem aeroben Prozeß hat so rasch wie möglich ein anaerober zu folgen, was durch Überpumpen und damit Eintauchen des Rottemistes durch Gärgülle aus dem Hauptgarbehälter erreicht wird, was die zweite anaerobe Phase einleitet. Sollte der Hauptgärbehälter eine derartige Entnahme aus Füllungsgründen nicht gestatten, kann ersatzweise auch Gärgülle aus dem Nachgärbehälter genommen werden. Rottemist, welcher über das Winterhalbjahr bis zu acht Monaten gelagert werden muß, kann erneut einem Wechsel zwischen anaeroben und aeroben Gärungsvorgängen unterzogen werden.

Die Erfahrung, welche ebenfalls in allen Betrieben verschieden sein wird, wird in Zukunft lehren, ob man einen solchen Wechselprozeß als sechste Stufe sinnvoll anwenden kann.

Der mehrstufige Prozeß erfüllt 3 Aufgaben:

1. Vollständiges Beseitigen der Umweltbelastung durch kontrollierte Gärungen

Belastungen der heutigen modernen landwirtschaftlichen Tierhaltung und der sich daraus ergebenden Düngerwirtschaft werden durch diese geordneten Gärungsvorgänge in biologische, unschädliche Formen überführt.

Weitverbreitete Geruchsbelästigungen durch Flüssigmist und falsche Gärgüllen werden hiermit beseitigt. Gleichzeitig werden damit auch die Emmissionen durch Nitratauswaschungen und anderen toxischen Stoffen aufgehoben, die sich bei falschen Gärungen im Bereich der Rhizosphäre -vor allem bei starken Düngungen mit frischen organischen Substanzen -für den Grundwasserbereich ergeben.

2. Verbindung von Flüssiggare mit Rottegärung

Hier werden durch Proteinsynthese und eingebaute Pilzkultur die erreichbaren möglichen Nährstoffmengen nicht nur erhalten, sondern zusätzlich vermehrt und in organische Formen gebracht.

3. Arbeitstechnischer Fortschritt

Für den mittleren und größeren landwirtschaftlichen Betrieb wird eine kompakte, überdachte und damit auch mehr oder weniger isolierte Anlage für die Rottegärung und die verschiedenen Temperaturbereiche erreicht. Diese bringt, ausgestattet mit modernen Greiferanlagen, Arbeitsersparnis und vor allem Arbeitserleichterung. Die Ausbringung auf Feldmieten mit Nährstoff-und Bodenverlusten erübrigt sich.

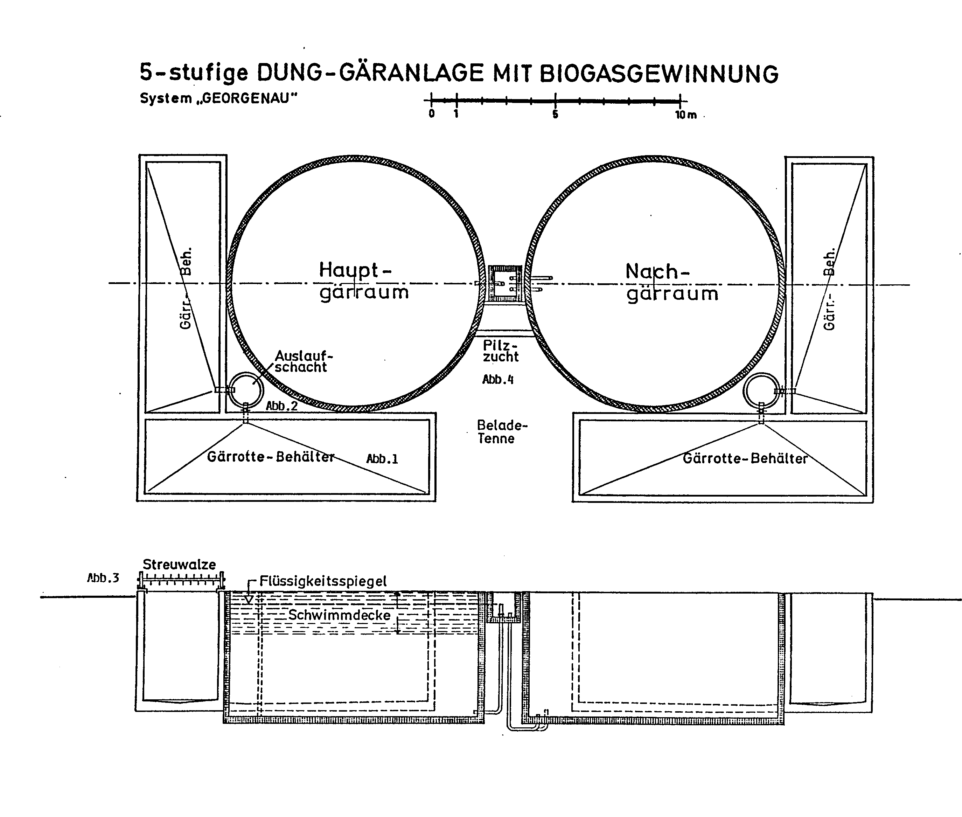

Ob in den Gärrottekammern (Abb.1) als Lagerbehälter mehr dem Gasauffang oder dem aeroben Wechselspiel der Vorrang gegeben wird, hängt auch von Betriebsstrukturen ab und muß zukünftig noch geklärt werden.

Die Wirtschaftlichkeit wird in betriebs-und volkswirtschaftlichen Nutzenberechnungen dargestellt.

Meine Patentanmeldung P 27 11 146.2 stellte auch einen Schritt in die neue Richtung dar und hatte mit
1. Verflüssigung
2. Trennung von Flüssigkeit und Feststoffen mit Schwimmdeckenentnahme und

3. Nachgärbehälter mit Gasauffang und Vorratschaltung auch schon eine 3-stufige Vergärung und Vorratschaltung dargestellt.

2.1. Die fünf Stufen des Verfahrens

Stufe I: Verflüssigung im Mischbehälter und Transport zum Hauptgärbehälter.
Stufe II: Trennung von Feststoffen und der flüssigen Gärgülle im Hauptgärbehälter durch Bildung einer Schwimmdecke.
Stufe III: Selbsttätiger Überlauf und Nachgärung im Nachgärbehälter, wobei sich bildende Methangase im schwimmenden Gasauffang und Vorratsbehälter gespeichert werden.
Stufe IV: Entfernung der Schwimmdecke alle ca. 6-8 Wochen durch Greiferaufzug und Zerkleinerung mit gleichzeitiger Durchsetzung von gezüchteten Pilzkulturen und Beimischung von Strohmehl oder Spreu zur aeroben -erwärmenden Rotte.
Stufe V: Anaerobie durch vollständiges Untertauchen d.h. Überpumpen mit Gärgülle aus dem Hauptgärbehälter -bei Mangel in Stufe I mit Nachgärgülle aus Stufe II.
Anhang zu V: Frage, ob eventuell als sechste Stufe ein Wechsel zwischen Aerobie und Anaerobie bei der Überwinterung in den Rottegärbehältern vorgenommen werden soll.

3. Patent-Anspruchsbegründung mit Angabe der technischen Merkmale des neuen Verfahrens

Die technische Neuheit der Verfahrens ist dadurch gekennzeichnet, daß zu den seitherigen zwei Gärbehältern, nämlich dem Hauptgärraum und dem Nachgärraum, mindestens vier Kammern als Gärrottebehälter angebaut werden. (Abb. 1) Diese Gärrottebehälter sind so angeordnet, daß sie für die beiden vorgenannten Gärbehälter gleichzeitig als Wärmeschutz und während des aeroben Rotteprozesses als Wärmequelle dienen.

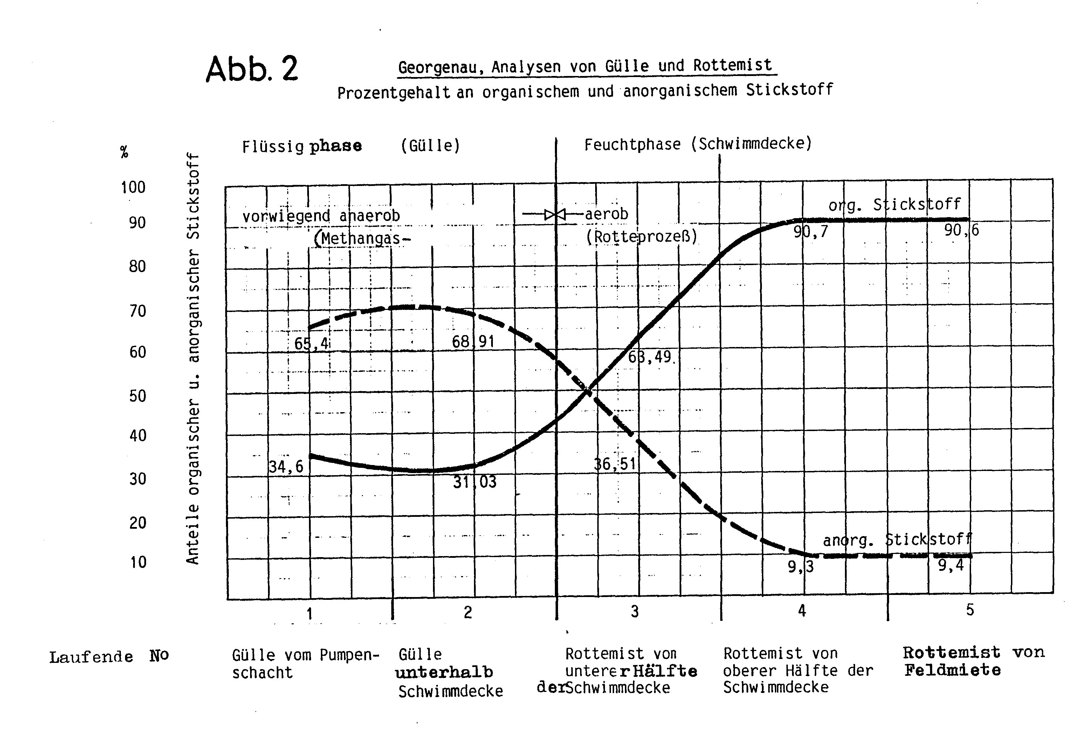

Diese Gärrottebehälter müssen in ihrem Volumen den Schwimmdeckenanfall von ca. 8 Wochen Gärzeit aufnehmen können. Für die im Verfahren beschriebenen Großanlagen mit mehreren 100 bis 1000 GV müssen bei starkem Strohanfall entsprechend mehr Gärrottebehälter erstellt werden, welche dann auch in der Lage sind, für den verkürzten Zeitraum von 4 bis 6 Wochen den anfallenden Schwimmdeckenmist aufzunehmen.
Die technische Ausstattung der Nachgärrottebehälter ist dadurch gekennzeichnet, daß jeder Gärrottebehälter am tiefsten Punkt eines leicht abgeschrägten Bodens einen Auslauf mit Schieber besitzt (Abb.2), welcher beim Überpumpen mit Gärgülle diese vor dem Auslaufen verhindert. Die Möglichkeit des Entfernens der Gärgülle zur Erhaltung eines normal-feuchten Festmistes wird durch die Anbringung von Sammelgruben, welche bei 1 bis 1,2 m Durchmesser dadurch gekennzeichnet sind, daß sie mindestens 0,5 m unter der Bodenfläche der Gärrottebehälter angebracht werden.

Aus diesen Sammelgruben kann während des Auslaufs mit einer kleinen elektrischen Pumpe die freiwerdende Gülle in den Überlaufschacht zum Nachgärbehälter befördert werden.

Die Kompaktheit der Gesamtanlage ist dadurch gekennzeichnet, daß sie durch die Anordnung der Gärrottebehälter um den Haupt-und Nachgärraum so angeordnet sind, daß damit der geringste Wärmeverlust für den Gesamtprozeß erreicht wird.

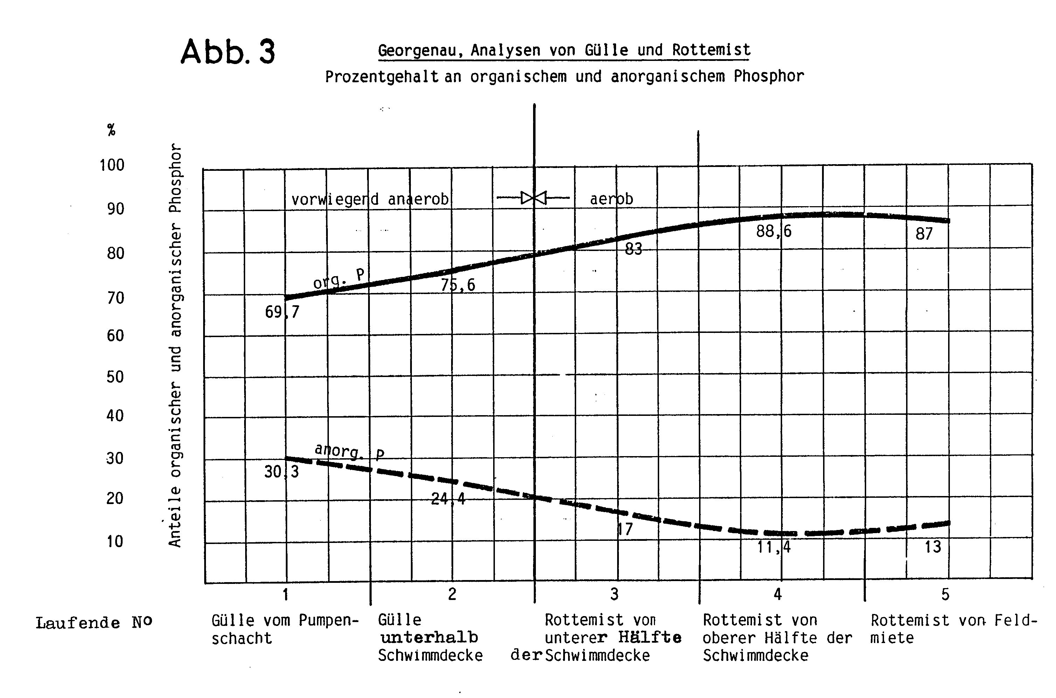

Die Mechanisierung für die Gärrottebehälter ist dadurch gekennzeichnet, daß mittels der Greiferanlage eine Streuwalze auf Schienen auf den oberen Rand angebracht und eingesetzt wird (Abb.3), damit im automaschinen Vor-und Rückwärtsbetrieb eine gleichmäßige Beschickung der Gärrottekammern mit zerkleinertem und damit belüfteten Schwimmdeckenmist erreicht wird.

Durch Zugabe von kleineren Mengen frischer organischer Substanz wird Selbsterwärmung im aeroben Prozeß erreicht und die Einbringung der Pilzkultur hiermit gleichzeitig vollzogen.

Die Möglichkeit zur Anzucht von geeigneter Pilzkultur ist dadurch gekennzeichnet, daß im innersten Freiraum der Anlage am Ende der Ladetenne und damit an der wärmegeschütztesten Stelle eine kleine Pilzzuchtkammer angelegt ist, welche mit ca. 2 bis 4 m³ Rauminhalt gute geeignete Schwimmdeckenmistkultur aufzunehmen vermag und sich vor dem Überlaufbehälter, zwischen Haupt-und Nachgärraum, befindet (Abb.4).

4. Die naturwissenschaftlichen Grundlagen als Anspruchsbegründung für ein Verfahrenspatent

Dieser Patentanmeldung zur Erreichung eines Verfahrenspatentes liegen meine beiden Patentanmeldungen P 27 11 146.2 vom 15.03.1977/Offenlegung am 28.09.1978, sowie die Zusatzanmeldung zu P 27 11 146.2, P 30 07 064.5 vom 26.02.1980/Offenlegung am 10.09.1981 zu Grunde, welche ich zurückgenommen habe, weil die Vergärung der Schwimmdecke nicht den erwarteten Erfolg aufwies und sich neue Gesichtspunkte aus der Erkenntnis der Mikrobiologie ergeben haben.

In einem 4-semestrigen Studium an der Universität Hohenheim als Gasthörer der Allgemeinen Agrarwissenschaften habe ich mir die Grundlagen für diese neue Patentanmeldung erarbeitet.

Die neue Erkenntnis beruht auf den in den letzten Jahren wissenschaftlichen Arbeitsergebnissen im Bereich der Mikrobiologie, inesbonsdere den Ergebnissen der Forschung in der Physiochemie und Mikrobiologie der Rhizosphäre. Prof.Dr. Ottow, der dieses Arbeitsgebiet leitet, bin ich zu großem Dank verpflichtet.

Prof.Dr. Ottow ist Präsident der Kommission für Bodenbiologie in der Deutschen und dritter Vizevorsitzender der Internationalen Bodenkundlichen Gesellschaft.

Das Ergebnis ist schlicht und einfach:

der Einbau von Pilzen in den Rotte-und Gärprozeß ermöglicht die Aufschließung von Lignin und Rohfaser.

An diesem Punkt scheiterte seither die Vergärung der Schwimmdecke.

5. Die verschiedenen Temperaturbereiche der Gärungsvorgänge

Raum und Zeit ergeben die Maßstäbe für die verschiedenen Temperaturbereiche, für die Größe des Hauptgärraumes und daran anschließend für die zur Schwimmdeckenentnahme notwendigen Rottegärbehälter. Die Temperaturhöhe ist maßgebend für die Zusammenhänge von Zeit und Raum, denn die Gasleistung und das Bakterienwachstum ( = Proteinbildung) sind zwei verschiedene Lebensäußerungen der Bakterienfloren. Man unterscheidet:

5.1. Physophiler Gärbereich (Vergärung ohne Beheizung)

Der physophile Gärbereich reicht von ca. 6°C, als untere Grenze mit dem dortigen Beginn des bakteriologischen Wachstums, bis 24°C. Dies ist der eigentliche Bereich der Bodengare, wo sich der wesentlichste Teil des Wachstums auf dieser Erde abspielt.

Bei der Bildung der Schwimmdecke ergibt sich ein neues Gärungsmilieu, welches die Flüssigkeit nach außen und unten verdrängt, indem es sich bei ca. 0,75 spez. Gewicht zu einem Viertel, bei sehr starkem Strohanteil und voller Vergärung bis zu einem Drittel, aus der Flüssigkeit heraushebt.

Bei der täglichen Belüftung durch den kontinuierlichen Beschickungsvorgang erfolgt jederzeit eine molekularcinetische Lufteintragung, welche beim Aufstieg in die Schwimmdecke nicht nur Sauerstoff sondern insbesondere auch annähernd 80% Luftstickstoff einbringt für die Tätigkeit der stickstoffsammelnden Bakterien.

Dieser Vorgang vollzieht sich entsprechend auch in den anderen Gärbereichen, ist aber im physophilen Bereich durch das stärkere Bakterienwachstum am ausgeprägtesten und benötigt dort nur mehr Zeit und Raum.

Beispiel: Gärraum für 1 GV in diesem Bereich liegt je nach Stroheinsatz, d.h. Belastung mit organ. Masse (Trockensubstanzmasse), bei

5-7 m³ Hauptgärraum (mit Gasauffangplane) und

4-6 m³ Nachgärraum (mit Gasspeicherplane),

da im Nachgärraum immer eine Resttiefe zum Schimmen des Gasvorratsbehälters verbleiben muß, ist die gleiche Größe des Nachgärraums anzustreben.

## 5.2. Mesophiler Gärbereich

Der mesophile (mittlere) Bereich von ca. 24-40°C bringt im Bereich der Körpertemperatur eine sehr hohe Wirkung in Hinsicht auf Energie = Methanbildung und auch auf Bakterienwachstum = Proteinbildung und dies bei wesentlich geringeren Raum-und Zeitverhältnissen.

Jedoch steht auf der Negativseite bei der Methanisierung:

1. Die Notwendigkeit des Beheizens mit technischem und Energieaufwand und

2. vor allem in kälteren Gegenden und Jahreszeiten der größere Energieverlust durch Wärmeabstrahlung von den Gärräumen und vor allem auf dem Beschickungsweg zwischen tierischem Körperaustritt und dem Gärraum.

Die antagonistische Wirkung dürfte nach vielen Erfahrungen bei dementsprechender längerer Zeit und größerem Raum im physophilen Bereich eher größer und wirksamer sein (z.B. Gärungen in Felsenkeller bei Käse-und Sektherstellung).

Dies näher zu ergründen ist Aufgabe der wissenschaftlichen Forschung. Wenn es in Zukunft gelingt, im Bereich von 36-39°C bei etwas erhöhten Zeit-und Raummaßen für den Schwimmdeckenbereich, die Wärmequelle aus Abwärme z.B. von Generatorenmotoren zu verwerten, so dürfte hier eine beachtliche Steigerung der Energie-und Wärmeausbeute und mit der Schwimmdecke und deren Nachgärung in den vier Rotte-Gärbehältern wohl auch der Proteinbildung liegen. Jedoch werden hierzu Mindestgrößen von ca. 40 GV notwendig sein.

Beispiel: Die ausgesprochenen Großbetriebe, wie sie bei uns nur einzeln vorhanden sind, und in den Ostblockstaaten als Sowchosen und Kolchosen üblich sind, müssen in diesen Temperaturbereichen betrieben werden.

Bei dort durchschnittlich 0,5 GV je ha LN Viehbesatz bedeutet dies bei 1000 ha ca. 500 GV je Betrieb. Bei einer Verweildauer von 25 Tagen im Hauptgärbehälter braucht man bei einem täglichen Dunganfall von 50 kg = Liter eine Gärraumgröße von 1,25 m³ je GV, d.h. für 500 GV eine Gärraumgröße von 625 m³. Dies entspräche einem Gärsilo von 10 m Durchmesser und 8 M Höhe.

Gärsilos in der Größe von 12 m Durchmesser und 12 m Höhe geben 1350 m³ Hauptgärraum und können somit bei gleichbleibenden Gärtemperaturen von 39°C den anfallenden Dung von 1000 GV vergären.

Kleinere Anlagen werden sinnvollerweise mit einer Verweildauer von 30 Tagen mit 1,5 m³ je GV gefahren, da größere Gärbehälter bei weniger Abstrahlungsverlusten durch die Wände einen etwas intensiveren Gärungsverlauf erreichen. Deshalb ist es sinnvoll, für kleinere Betriebe die Verweildauer von 25 auf 30 Tage zu verlängern, d.h. die Gärraumgröße je GV muß anstatt mit 1,25 m³ mit 1,5 m³ angesetzt werden.

## 5.3. Hochgärtemperaturbereich

Der Hochgärtemperaturbereich von 50-59°C hat sich in der Praxis als sehr schwierig und unzuverlässig erwiesen; in diesem Pasteurbereich kann man wohl kurzfristig auftretende Epidemien - schneller bewältigen, jedoch man verzichtet auf die antagonistische Wirkung der beiden Gärbereiche, welche im physophilen Bereich dem Wesen der Bodengare und im mesophilen Bereich der Verdauung im tierischen Körper entspricht.

Hieraus ergibt sich die Möglichkeit in mittleren und vor allem größeren Anlagen im Notfall z.B. bei Maul-und Klauenseuche, Schweinepest, Hühnerpest u.ä., Salmonellen u. dgl. eine Bekämpfung dieser Seuchen durch Erhitzung in den Pasteurbereich vorzunehmen.

Bei klarer, sauberer Führung in den beiden Naturbereichen der physophilen und der mesophilen Gärung werden sich solche Maßnahmen weitestgehend erübrigen.

Dies zeigte mit aller Deutlichkeit eine Maul-und Klauenseuche in der Schweiz Anfang der 60er Jahre, bei der nach Mitteilung von Dr. Hans Müller kein einziger der über 400 organ.-biologisch bewirtschafteten Betreibe von der Seuche betroffen war.

Die Rottegärbehälter für die Schwimmdecke werden nach Stroheinsatz und Einbringen von sonstigen organischen Substanzen (Gras, Laub, Spreu u.s.w.) in jedem Betrieb je nach Wirtschaftsweise verschieden sein und werden sich um einen Mittelwert je GV von 1,25 bis 1,5 $m^3$ der einzelnen Gärottebehälter (4 Stück) bewegen.

Diese werden um die beiden Haupt-und Nachgärbehälter in der Bauausführung angeordnet und bilden gleichzeitig einen Wärmeschutz.

Bei 8-wöchiger Schwimmdeckenentnahme würde dies für den ganzen Jahresablauf eine Entleerung in 6 Gärrottebehälter bedeuten. Da aber im Verlauf des Jahres während der Vegetationszeit von Mai bis Ende Oktober die Zeit für die Ausbringung vor allem auf abgeerntete Flächen gegeben ist, reichen bei 6 Gärrottebefüllungen 4 solcher Kammern für einen kontinuierlichen Betrieb .

Um am Beispiel des Großbehälters für 1000 GV diesen Verfahrensablauf aufzuzeigen, müßte bei der raschen Zunahme der Schwimmdecke dort die Abtragung derselben mindestens alle 4 Wochen erfolgen. Es sind also mindestens 6 solcher Kammern notwendig, um neben der Vergärung vor allen Dingen im Winterhalbjahr keine Ausbringung vornehmen zu müßen.

Für alle Anlagen, die überdacht werden und damit auch Greiferanlagen zur Entnahme besitzen, kann man in jedem Betrieb Regentage für diese Aufgabe verwenden, wo sonst auf dem Feld keine andere sinnvolle Tätigkeit ausgeführt werden kann.

6. Betriebs-und volkswirtschaftliche Nutzenberechnungen

6.1. Wirtschaftlichkeit

6.1.1. Ausgangssituation

Die Wirtschaftlichkeit für den Einzelbetrieb läßt sich nur anhand von Mittelwerten berechnen, wobei dieselben je nach Betriebsgröße und Intensitätsgrad verschieden sind.

Ausgegangen wird von einer bäuerlichen Betriebsstruktur, welche eine angemessene technische Austattung besitzt. Landwirtschaftliche Kleinstbetriebe gehören zur Hobbygärtnerei und bleiben bei dieser Aufgabestellung außer Betracht, für sie muß diese Aufgabe in genossenschaftlicher Form durchgeführt werden.

Untere Grenzen liegen: Bei 20-40 GV, d.h. bei 10-30 ha LN.

Der Durchschnitt dürfte bei 40-60 GV, d.h. bei 30-60 ha LN liegen.

Die oberen Grenzen liegen bei 100-200 GV, das entspricht 60-200 ha LN.

Großanlagen liegen bei ca. 1000 GV.

6.1.2. Nährstoffbilanz

Der Vergleich eines alternativ bewirtschafteten Betriebes mit einem Betrieb mit hohem Mineraldüngeraufwand . soll auf der Dünger-Nährstoffbasis eines konventionell bewirtschafteten Betriebes errechnet werden.

Bei einem durchschnittlichen Getreideertrag als Grundmaß von 45-60 dt je ha LN Getreide, als Maßstab auch für Zuckerrüben, Raps und ähnliche Ackerfrüchte.

Der alternativ wirtschaftende Betrieb mit durchschnittlich 1 bis 1,4 GV je ha LN schließt mit dem natürlichen Düngeranfall (wobei die Rinderhaltung nicht fehlen darf) seinen Nährstoffkreislauf, ohne Zukauf von Mineraldünger (N, $P_2O_5$, $K_2O$).

Eine GV je ha entspricht in etwa dem Tierbestend der BR Deutschland bei 12 Mill. ha LN.

Nach Erfahrungen und Meßungen im Exaktdüngungsversuch auf meinem Pachtbetrieb in Untersontheim, Kreis Schwäbisch Hall durch das Landwirtschaftsamt Schäbisch Hall 1955 und mit Untersuchungen der Chemischen Untersuchungsanstalt der Universität Hohenheim ergaben sich folgende Nährstoffmengen:

Bei 1 GV = ca. 135 dt Frischmist und 7 $m^3$ Jauche.

Bei der Biovergärung wurden ca. 100-110 dt Schwimmdeckenmist entnommen (der Rest, bis 135 dt, entspricht einem ca. 30%igen Abbau an organischer Substanz.

Nach Methanvergärung und aerober Nachrotte ergab dies je GV und Jahr folgende Nährstoffmengen, welche voll wirksam waren

(Hinweis: siehe Neuauflage von "Neue Erkenntnisse in der Düngerwirtschaft".) :

(Konventionell nach Prof.Dr.Dr.hc von Boguslawski und Dr. Debruck, Gießen -"die Ausnutzung der mit organischen Substanzen zugeführten Nährstoffe" -, in Menzel 1973, S.355).

| | N | $P_2O_5$ | $K_2O$ | |
|---|---|---|---|---|
| biologisch | 72,8 | 31,2 | 58,0 | kg |
| konventionell | | | | |
|     Anfall insgesamt | 60,0 | 22,2 | 60,0 | kg |
|     davon wirksam | 20,0 | 13,0 | 48,0 | kg |
| | | | | |
| Differenz | 52,8 | 18,2 | 10,0 | kg |
| Wert je kg in DM | 2.- | 2.30 | 1.- | |
| Wert insgesamt in DM | 105.60 | 41.80 | 10.00 | |

Wertdifferenz:    157.40 DM pro GV und Jahr

---

1) Hinweis: siehe Neuauflage von "Neue Erkenntnisse in der Düngerwirtschaft".

Beim Exaktdüngungsversuch in 4-facher Wiederholung und bei Nährstoffgleichheit ergab sich bei Kartoffeln ein Mehrertrag von 19% und im folgenden Jahr bei Weizen nochmals ein Mehrertrag von 6% bei einem Körner-Stroh-Verhältnis von 106 zu 104.

Der Mehrertrag und die Beseitigung der Emmissionen (Geruch und Auswaschungen), sowie Flächenersparnis zur Kompostierung und auch die daraus zu erzielende Arbeitsersparnis dürften zusammen nochmals mit 150.-DM je GV und Jahr angesetzt werden.

Insgesamt ergibt sich daraus ein Betrag von 300.-DM je GV und Jahr.

Der biologische Mehrwert der Erzeugnisse, sowie der Gaswert, welcher bei 0,8 m³ Bio-Normalgas (ca. 56% Methananteil, Rest vorwiegend $CO_2$) und einem Heizölpreis von 0,60 DM je Liter = 102 DM jährlich betragen würde, bleiben hier außer Betracht.

Der Gaswert muß dem Kostenaufwand für Gasauffang und Gasspeicherung, sowie Verwertung decken und bleibt eine technische, absolut lösbare und auch wirtschaftlich interessante Aufgabe für die nächsten Jahre.

Kosten für eine Anlage für 100 GV:

Bauliche Anlage ca.    200 000 DM
Technische Anlage ca.   60 000 DM
_____

Zus.              260 000 DM
                 ==========

Jährliche Kostenbelastung:

Bei 2% Amortisation für die bauliche Anlage   =    4 000 DM

und 10% Amortisation für die technische Anlage =    6 000 DM

und 5,5%ige Verzinsung (von 260 000 DM)     =    14 300 DM
_____

jährliche Kostenbelastung insgesamt         24 300 DM
                                   ==========

Dem gegenüber steht ein Nutzen (bei 100 GV) von 30 000 DM ohne Gaswert!! Wenn man die jährliche Kostenbelastung noch auf 25 000 DM aufrundet (sonstige anfallende Kosten, wie z.B. Versicherungen) bleibt ein Gewinn von 5 000 DM pro Jahr

Der eigentliche Gewinn ergibt sich in erhöhtem Maße für die alternative Landbewirtschaftung, welcher mit Sicherheit die Zukunft gehört.

6.2. Volkswirtschaftlicher Wert

Der volkswirtschaftliche Wert, bei dem man den Gaswert ohne weiteres mit einbeziehen kann, beträgt bei der Annahme von 12 Mill. ha LN und 1 GV pro ha und einem Nutzen von 400 DM je GV (einschl. 100 DM Gaswert):

4,8 Mrd. DM

Das sind ca. 13,3% der heutigen Bruttowertschöpfung der Landwirtschaft in der BR Deutschland.

Vom Produktionswert der Landwirtschaft, einschließlich der Vorleistungen, wären es ca. 7%.

Der genauere Maßstab wären allerdings 13% der Bruttowertschöpfung.

In allen geordneten Regelkreisen und Gärungsvorgängen vollzieht sich eine positive Energie-und Humusbilanz. Dort werden nicht wie in den heutigen modernen Industrieanlagen die Ressourcen dieser Erde abgebaut, ausgebeutet, mit Belastungsfolgen von Schwermetallen, Strahlungsschäden und Giftschäden.

Dort werden, dem ewigen "Es werde" dienend, Schwermetalle in den biologischen Prozessen als naturnotwendig wieder eingebaut.

Wenn man den höheren biologischen Wert der gesamten Agrarproduktion und die Beseitigung von Geruchs-und Wasseremmissionen noch zusätzlich ansetzt, so kommt man auf einen volkswirtschaftlichen Wert, der mindestens mit 5 Mrd. DM Wertschöpfung auszuweisen ist.

Das macht 8% des gesamten Produktionswertes der Deutschen Landwirschaft aus.

7. Ökologische Folgerungen

7.1. Die Korrektur der Lehrmeinung in der Agrikulturchemie

Die Lehrmeinung der Agrikulturchemie, die bis heute auf der Grundlage der Liebig schen Erkenntnisse aufgebaut ist, beinhaltet folgende Schwächen:

Justus von Liebig sah auch noch selbst im Alter in einer seiner letzten Veröffentlichungen, dem 23. Chem. Brief "Über den Materialismus" - (herausgegeben vom Forschungsring für bioldynamische Wirtschaftsweise durch Dr. Hans Heinze, in unveränderter Schreibweise von 1865), den Gesamtvorgang folgendermaßen:

"...der Lebensprozeß der Pflanze ist der Gegensatz des Oxidationsprozesses, der in der anorganischen Natur vor sich geht, er ist ein Reduktionsprozess."

Justus von Liebig können wir heutigen Zeitgenossen keinen Vorwurf machen, da ihm noch nicht die Ergebnisse der Naturwissenschaften auf dem Gebiet der Mikrobiologie zur Verfügung standen.

Er sah nur die eine seite des Gesamtredoxpotentials, nämlich den Ablauf durch Oxidation (Verbrennungsprozeß), wo beim Abbau von einer Einheit organischen Kohlenstoffs ca. 417 cal. Wärme frei werden, während wir heute wissen, daß bei der anderen Seite der Reduktionsvorgänge, nämlich in allen Gärungsbereichen der Natur (Boden, Hefe, Brot, Verdauungsvorgänge, Alkohol u. ä. Gärungen) in der höchsten Reduktionsstufe zu Kohlenwasserstoff, nämlich im Methangas - ($CH_4$) dieselbe Menge Energie freigesetzt wird. Dabei steht uns in Form von Gasen oder auch ähnlichen Flüssigkeiten (Methanol, Ethanol) dieselbe Menge Energie zur Verfügung.

Allerdings dann in Gasform oder als Flüssigkeit, welche wir Menschen heute im Zeitalter der Technik dann disponierbar ansammeln und einsetzen können, um in Verbrennungsvorgängen annähernd dieselbe Energie dann zu Verfügung zu bekommen.

Die vollendetste Form eines solchen Gärungsvorganges gibt uns die Natur in dem Vorbild des Rinderverdauungsapparates und -prozesses.

Nachdem der Oxidationsprozess vorwiegend zur Mineralisation führt, weshalb die heute gültige Lehrmeinung in der Pflanzenernährung und Düngerwirtschaft einseitig nur diese Seite des Redoxpotentials erkannt und als Mineralisation angewandt hat.

Die Mineralisationstheorie wurde am klarsten und eindeutigsten in der bildhaften Darstellung des Gesetzes vom Minimum als Grundlage der Pflanzenernährung richtig dargestellt.

Es gilt heute die Ganzheit der Naturvorgänge richtig zu sehen und sinnvoll anzuwenden. Nämlich das Bestreben der Natur die Mineralisation zu überwinden und zu überführen in organische, biologisch vollvertige Verbindungen. Bei der Bildung von Proteinen und lebendigen Kohlenhydratverbindungen, wie auch im Gesamtbereich der Hormone, Vitamine und ähnlicher Wirkstoffe werden dann gleichzeitig auch die Schwermetalle (Aluminium, Cadmium, Blei u.s.w.) wieder in den Naturbereich eingebaut.

Dann werden diese Erscheinungen aus ihrer derzeitigen Gefährlichkeit aus unserer falsch eingesetzten Technologie des Industriezeitalters wieder zurückgeführt in den eigentlichen Naturbereich und werden in unseren Böden bei allen humusbildenden Vorgängen wieder ihrer natürlichen Zweckdienlichkeit zugeführt.

Die Nährstoffe werden wie Exaktversuche beweisen und die Wissenschaft heute weiß nicht nur erhalten, sondern bei Stickstoff und Phosphat zusätzlich vermehrt durch Proteinsynthese.

Solche Anlagen dienen in höchstem Maße der Volksgesundheit und geben die Möglichkeit, die heute immer mehr ansteigenden Sozialkosten auf dem Gesundheitssektor zu dämpfen.

7.2. Raum, Zeit, Gewicht und Energiebilanz mit dem daraus resultierenden Transportproblem

Die sinnvolle Rückführung aller organischen Stoffe in den Naturkreislauf gestattet deshalb nicht mehr, Stroh, Spreu und andere organische Substanzen dem gewöhnlichen willkürlichen Rottevorgang an der Oberfläche oder beim Einarbeiten - (Stoppelschälen u.s.w.) zu überlassen, oder gar noch zu verbrennen, weil jeder Verrottungsvorgang jedes einzelnen Strohhalms dem umgebenden Boden zuerst Stickstoff entzieht, um in der ersten Verrottungs -Fäulnisphase zwangsläufig auch mehr oder weniger starke toxische Wirkungen zu erzeugen und damit allen Gefahren der Denitrifikation und Auswaschungen willkürlich zu unterliegen.

Daraus resultiert: dieser ganze Vorgang einer vollständigen Rückführung und geordneten geschlossenen Gärung stellt ein sehr beachtliches Transportproblem dar. Dies stellt natürlich die logische Frage, ob ein solches Verfahren überhaupt ökonomisch sinnvoll sein kann.

Zur Problemdarstellung: Wenn Teile wie Stroh, Spreu, Blattabfälle auf dem Feld verbleiben um Transport zu sparen, so muß unbedingt so viel aufgeschlossene Düngersubstanz, am Besten endverrotteter Schwimmdeckenmist oder gut vergorene Gärgülle, dazu gebracht werden, damit der biologische Umsetzungsprozess diesen starken Strohanteil verarbeiten kann.

Eine volle Strohernte so einzuarbeiten würde je ha mindestens 100 dt Schwimmdeckenmist und 10 m³ Gärgülle bedingen.

Dies wäre eine doppelte Düngung und dürfte für das folgende Nutzungsjahr nur zu Starkzehrern wie Hackfrüchte, Mais und dgl. gegeben werden.

Damit wird der Beweis erbracht, daß eine ökologische Landbewirtschaftungsweise niemals mehr zu Großbetrieben als Ideal führen wird, wie dies in der heutigen Tendenz des "Wachsen und Weichen" geführt hat, wo die Auswirkungen in den Naturvorgängen nicht mehr mit einer wahrhaften Ökonomie übereinstimmen.

Denn ökonomisch wirtschaften, heißt in Wirklichkeit, sinnvoll und sparsam wirtschaften, wie die Natur es uns lehrt.

Die wahre Ökonomie kann niemals im Gegensatz zu den Naturvorgängen stehen, welche uns im Gärungsbereich der Rhizosphäre und der Verdauungsvorgänge von der Natur vorgezeichnet sind.

7.3. Der Irrweg des Sozialismus einer technologisierten Landwirtschaft in Sowchosen und Kolchosen

Aus diesen Gesichtspunkten ergibt sich auch das naturwidrige, ökonomische Denken, welches in der technologisierten Landwirtschaft des Sozialismus durch Lenin und seine Zeit und Glaubensgenossen eingeführt wurde.

Es ergibt sich wieder die jahrtausendalte Erfahrung, daß die günstigste Form der Betriebsgröße in der einstigen Königshufe von 50 ha liegt (im Idealfall ca. 700 x 700 m).

Dies ist die am wenigsten energiebedürftige Form für die Transportaufgaben der Einsammlung, Vergärung und Ausbringung aller organischen Substanzen.

Die günstigste Schlaglänge liegt bei ca. 300 m für Dungausbringung und Ernteeinbringung. Die Natur hat eben immer ihre eigenen optimalen Größen und es ist erstaunlich, daß eine solche, wie hier vorgezeichnete Betriebsweise, sich wieder mit den überlieferten Formen und Größen in unserem europäischen Bauernleben deckt.

Niemand, auch kein noch so gut durchdachtes technisches System, kann sich über diese Naturgegebenheiten hinwegsetzen. Die Entwicklung führt also zwangsläufig zu dem Bestreben mit Hilfe der Technik der Natur zu dienen. und nicht umgekehrt. Dies führt zu ganz neuen Gesichtspunkten im technischen, wie im ökonomischen Denken.

Dies soll in dem Satz zum Ausdruck gebracht werden:

"Nicht Landwirtschaft im Zeitalter der Technik, sondern Technik im Dienst der Natur."

9. Literaturverzeichnis und Zeichnungen

1. Neue Erkenntinisse in der Düngerwirtschaft -Neue Wege zum alternativen Landbau-Ein Beitrag zu ökologischen Umweltschutz und zum Energieproblem, Fritz Weber, Hof Georgenau 1977, erweiterte Auflage 1979.

2. zum Stand der Technik:

Offenlegungsschrift 27 11 146

3. "Über den Materialismus", Justus von Liebig, Schriftenreihe Lebendige Erde

4. Zeichnung zur technischen Neuheit mit Draufsicht und Querschnitt

5. Graphische Darstellung über Umwandlung der Nährstoffe in organische Formen

**Ansprüche**

3. Patent-Anspruchsbegründung mit Angabe der technischen Merkmale des neuen Verfahrens

Die technische Neuheit der Verfahrens ist dadurch gekennzeichnet, daß zu den seitherigen zwei Gärbehältern, nämlich dem Hauptgärraum und dem Nachgärraum, mindestens vier Kammern als Gärrottebehälter angebaut werden. (Abb. 1) Diese Gärrottebehälter sind so angeordnet, daß sie für die beiden vorgenannten Gärbehälter gleichzeitig als Wärmeschutz und während des aeroben Rotteprozesses als Wärmequelle dienen.

Diese Gärrottebehälter müssen in ihrem Volumen den Schwimmdeckenanfall von ca. 8 Wochen Gärzeit aufnehmen können. Für die im Verfahren beschriebenen Großanlagen mit mehreren 100 bis 1000 GV müssen bei starkem Strohanfall entsprechend mehr Gärrottebehälter erstellt werden, welche dann auch in der Lage sind, für den verkürzten Zeitraum von 4 bis 6 Wochen den anfallenden Schwimmdeckenmist aufzunehmen.

Die technische Ausstattung der Nachgärrottebehälter ist dadurch gekennzeichnet, daß jeder Gärrottebehälter am tiefsten Punkt eines leicht abgeschrägten Bodens einen Auslauf mit Schieber besitzt (Abb.2), welcher beim Überpumpen mit Gärgülle diese vor dem Auslaufen verhindert. Die Möglichkeit des Entfernens der Gärgülle zur Erhaltung eines normal-feuchten Festmistes wird durch die Anbringung von Sammelgruben, welche bei 1 bis 1,2 m Durchmesser dadurch gekennzeichnet sind, daß sie mindestens 0,5 m unter der Bodenfläche der Gärrottebehälter angebracht werden.

Aus diesen Sammelgruben kann während des Auslaufs mit einer kleinen elektrischen Pumpe die freiwerdende Gülle in den Überlaufschacht zum Nachgärbehälter befördert werden.

Die Kompaktheit der Gesamtanlage ist dadurch gekennzeichnet, daß sie durch die Anordnung der Gärrottebehälter um den Haupt-und Nachgärraum so angeordnet sind, daß damit der geringste Wärmeverlust für den Gesamtprozeß erreicht wird.

Die Mechanisierung für die Gärrottebehälter ist dadurch gekennzeichnet, daß mittels der Greiferanlage eine Streuwalze auf Schienen auf den oberen Rand angebracht und eingesetzt wird (Abb.3), damit im automaschinen Vor-und Rückwärtsbetrieb eine gleichmäßige Beschickung der Gärrottekammern mit zerkleinertem und damit belüfteten Schwimmdeckenmist erreicht wird.

Durch Zugabe von kleineren Mengen frischer organischer Substanz wird Selbsterwärmung im aeroben Prozeß erreicht und die Einbringung der Pilzkultur hiermit gleichzeitig vollzogen.

Die Möglichkeit zur Anzucht von geeigneter Pilzkultur ist dadurch gekennzeichnet, daß im innersten Freiraum der Anlage am Ende der Ladetenne und damit an der wärmegeschütztesten Stelle eine kleine Pilzzuchtkammer angelegt ist, welche mit ca. 2 bis 4 m³ Rauminhalt gute geeignete Schwimmdeckenmistkultur aufzunehmen vermag und sich vor dem Überlaufbehälter, zwischen Haupt-und Nachgärraum, befindet (Abb.4).

# 5-stufige DUNG-GÄRANLAGE MIT BIOGASGEWINNUNG

System „GEORGENAU"

0 1     5     10 m

Gärr.- Beh.

Hauptgärraum

Nachgärraum

Gärr.- Beh.

Auslaufschacht

Pilzzucht

Abb.4

Abb.2

Belade-
Tenne

Gärrotte-Behälter    Abb.1

Gärrotte-Behälter

Streuwalze

Abb.3

Flüssigkeitsspiegel

Schwimmdecke

0 214 320

**Abb. 2**  Georgenau, Analysen von Gülle und Rottemist
Prozentgehalt an organischem und anorganischem Stickstoff

Flüssigphase (Gülle)  —  Feuchtphase (Schwimmdecke)

vorwiegend anaerob  —  aerob (Rotteprozeß)
(Methangasorg. Stickstoff
90,7 — 90,6

65,4 — 68,91 — 63,49

34,6 — 31,03 — 36,51

anorg. Stickstoff
9,3 — 9,4

% — Anteile organischer u. anorganischer Stickstoff

Laufende No

1 — Gülle vom Pumpenschacht
2 — Gülle unterhalb Schwimmdecke
3 — Rottemist von unterer Hälfte der Schwimmdecke
4 — Rottemist von oberer Hälfte der Schwimmdecke
5 — **Rottemist von Feldmiete**

0 214 320

# Abb. 3

Georgenau, Analysen von Gülle und Rottemist

Prozentgehalt an organischem und anorganischem Phosphor

Anteile organischer und anorganischer Phosphor

vorwiegend anaerob ←▷◁→ aerob

org. P
69,7   75,6   83   88,6   87

anorg. P
30,3   24,4   17   11,4   13

Laufende No

| 1 | 2 | 3 | 4 | 5 |
|---|---|---|---|---|
| Gülle vom Pumpen-schacht | Gülle **unterhalb** Schwimmdecke | Rottemist von **unterer Hälfte** der Schwimmdecke | Rottemist von oberer Hälfte der Schwimmdecke | Rottemist von Feld-miete |

0 214 320

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | AT-B- 361 015 (WEYMELKA WALTER) <br> * Figuren; Patentansprüche 1,4,5; Seite 3, Zeilen 17-20 * <br><br> --- | S.7, Z.3-17; S.8, Z.1-4; S.8, Z.16-23 | **C 12 M 1/00** |
| A | FR-A-2 419 322 (A- BETONG AB) <br><br> * Figuren 4,5; Seite 8, Zeilen 29-34 * <br><br> --- | S.7, Z.3-17 | |
| A | US-A-3 954 619 (L.J. FRY) <br><br> * Figuren * <br><br> --- | S.8, Z.5-15 | |
| Y | DE-A-3 327 541 (W. WISSUSEK) <br><br> * Figuren; Patentansprüche 1,2,7.10,16; Seite 29, Zeilen 11-29 * <br><br> --- | S.7, Z.3-17; S.8, Z.1-4; S.8, Z.5-15 | RECHERCHIERTE SACHGEBIETE (Int Cl 4) <br><br> C 12 M |
| Y | DE-A-2 711 146 (WEBER FRITZ) <br><br> * Figuren * <br><br> --- | S.7, Z.1-17; S.8, Z.1-4, 5-15, 16-23 | |
| A | GB-A- 621 747 (G.L.R. DUCELLIER) <br><br> --- -/- | | |

Der vorliegende Recherchenbericht wurde für alle Patentansprüche erstellt

| Recherchenort <br> DEN HAAG | Abschlußdatum der Recherche <br> 03-06-1986 | Prüfer <br> COUCKE A.O.M. |
|---|---|---|

Europäisches
Patentamt

**EUROPÄISCHER RECHERCHENBERICHT**

Nummer der Anmeldung

EP 85 11 1028

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Ci 4) |
|---|---|---|---|
| A | BE-A- 894 086 (M. HUNZIKER et al.) <br> * Figuren; Patentansprüche 1,6,8,9; Seite 6, Zeile 29 - Seite 7, Zeile 26 * | S.7, Z.3-17 | |
| A | US-A-4 053 395 (H. SWITZGABE) <br><br> * Figuren; Spalte 4, Zeilen 57-64 * | S.8, Z.5-15 | |
| A | BE-A- 470 761 (G. PETROES) | | |

RECHERCHIERTE SACHGEBIETE (Int Ci 4)

Der vorliegende Recherchenbericht wurde für alle Patentansprüche erstellt

| Recherchenort <br> DEN HAAG | Abschlußdatum der Recherche <br> 03-06-1986 | Prüfer <br> COUCKE A.O.M. |
|---|---|---|